# EUROPEAN PATENT SPECIFICATION

(11) **EP 3 013 391 B1**
(45) Date of publication and mention of the grant of the patent: **24.10.2018**
(21) Application number: 14744993.8
(22) Date of filing: 25.06.2014
(51) Int. Cl.: A61M 5/168, A61M 5/142, A61M 5/145, C25B 11/04, C25B 15/08, C25B 1/04

(54) **ELECTROLYSIS RECOMBINATION CONTROL MASK AND METHOD OF MANUFACTURE THEREOF**
ELEKTROLYSEREKOMBINATIONSSTEUERUNGSMASKE UND VERFAHREN ZUR HERSTELLUNG DAVON
MASQUE ANTI-RECOMBINAISON POUR ÉLECTROLYSE ET SON PROCÉDÉ DE FABRICATION

(30) Priority: 25.06.2013 US 201361839166 P
(43) Date of publication of application: 04.05.2016
(73) Proprietor: MiniPumps, LLC, Pasadena, Californa 91107 (US)
(72) Inventor: DUNN, Andrew, Santa Monica, CA 90403-4334 (US); SHIH, Jason, Yorba Linda, CA 92886-4574 (US)
(74) Representative: Somervell, Thomas Richard
(86) International application number: PCT/US2014/044106
(87) International publication number: WO 2014/210161

(56) References cited:
- WO-A1-93/07920
- WO-A1-96/04953
- WO-A1-2011/022484
- WO-A2-2007/106557
- DE-A1- 19 756 775

## Description

### CROSS-REFERENCE TO RELATED APPLICATIONS

The present application claims priority to, and the benefits of, U.S. Serial No. 61/839,166, filed on June 25, 2013.

### FIELD OF THE INVENTION

In various embodiments, the present invention relates generally to electrode fabrication and, more specifically, to fabrication of electrodes for electrolytic pump devices.

### BACKGROUND

Electrolytic pumps use electrochemically generated gases as a source of pressure that is used to dispense fluid (e.g., medicament) from one location to another. For example, application of a suitable voltage across two metal electrodes (e.g., platinum, gold, or palladium) immersed in an aqueous electrolyte produces oxygen and hydrogen gases that can apply pressure to a piston, membrane, diaphragm, or other force transducer. Electrolysis of water occurs rapidly and reversibly in the presence of a recombination catalyst such as platinum, which in the absence of an applied voltage catalyzes recombination of the hydrogen and oxygen to reform water. Electrolysis mechanisms may be advantageously used for drug delivery, as they can be electronically controlled and the electrolysis and drug reservoirs refilled.

Electrolytic pumps offer several advantages for drug-delivery applications. Their low-temperature, low-voltage and low-power operation makes them well-suited for long-term operation in vivo. For ocular applications, electrolytic pumps advantageously produce negligible heat, and can also achieve high stress-strain relationships. Additionally, the gas evolution proceeds even in a pressurized environment (e.g., 300 MPa) and produces oxygen and hydrogen gases that contribute to a volume expansion of about a thousand times greater than that of the electrolyte (e.g., water) used in the reaction. Moreover, they lend themselves readily to the use of microelectronics to control the voltage and current applied to the pump (and therefore the temporal pattern of pressure generation).

Nevertheless, electrolytic pumps generally require application-specific manufacturing. The electrode configuration and patterning, for example, can be designed or altered to accommodate different pumping requirements that in turn translate into voltage, current, and recombination requirements. Electrode efficiency is further affected by electrode material, geometry and surface conditions.

The overall efficiency of electrolysis devices is also affected by system-level parameters such as the pump size, drug reservoir size, drug reservoir shape, electrolyte mixture characteristics, cracking pressures of check valves fluidically connected to the drug reservoir, and ambient pressures that the pump may experience during use. These secondary factors indirectly affect the requirements of the electrolysis electrodes.

The electrodes can be patterned in varying shapes. A simple configuration, for example, consists of two flat electrodes that are inserted into the drug chamber. More elaborate patterns have the electrodes shaped as parallel rods, parallel wires, coaxial members, etc. The electrodes may also be patterned onto a surface to increase the surface area exposed. Furthermore, multiple pairs of electrodes may be used for purposes of redundancy.

Implantable medical devices have carefully budgeted power requirements due to limited space for a battery, compliance issues related to charging, and/or the costs associated with explanting a non-rechargeable device. As a result, meticulous calculations and iterative testing are typically performed to ensure that application-specific electrode configurations meet the power requirements of the device. Parameters such as electrolysis gas generation speed, recombination speed and current draw may be tuned through iterative modification of electrode materials, spacing, shape, width etc. Currently, however, this iterative procedure involves construction of finished devices, each of which is tested and modified for the next iteration. There is currently no practical way to modify or vary an already-fabricated electrode pattern for testing and further modification.

### SUMMARY

Embodiments of the present invention provide a recombination mask integrated onto an electrolysis chip to tune electrolysis parameters by masking off portions of the electrodes exposed to the electrolyte solution. This prevents electrolysis gases from reaching the catalyst on the electrodes and recombining. Masking off areas of electrodes made of catalytic material such as platinum slows the recombination of those gases back into electrolyte, thereby affording control over the electrolysis rate - in particular, over the ratio of the rate of gas generation to the rate of gas recombination, which represents a critical parameter for pump performance. If too much gas recombines at a given electrolysis current, pumping comes to a halt, and a greater electrolysis current is required to promote gas generation and actuate drug delivery.

In some embodiments, the electrodes are distinct members spaced apart from each other. In such configurations, both electrodes may be masked symmetrically (by a single mask or by a mask on each electrode) or a single electrode can be masked; the operative effect will be equivalent.

Accordingly, in a first aspect, the invention pertains to a device for administering a liquid. In various embodiments, the device comprises a housing; within the housing, a pump assembly including a reservoir, an electrolytic forcing mechanism and a cannula for conducting liquid from the reservoir to an ejection site exterior to the housing in response to pressure applied by the forcing mechanism; and internal to the forcing mechanism, an electrolyte reservoir and, therein, an electrode assembly comprising (i) at least two electrodes and (ii) over a portion of at least one of the electrodes, a gas-impermeable mask.

The mask may be sized and shaped so that the ratio of masked to unmasked electrode portions achieves a target operating ratio of gas recombination to gas generation. In typical implementations, the electrodes are disposed on an electrolysis chip. The electrodes may comprise or consist essentially of at least one of platinum, gold, or silver on parylene, ceramic, or a biocompatible insulator. Thus, the electrodes may act as a recombination catalyst, but additional recombination catalyst may be added to the electrolyte reservoir to augment recombination.

In some embodiments, at least a portion of the mask structure is bonded to the electrodes with an epoxy. The mask structure may comprise or consist essentially of PEEK, ceramic, aluminum. A spacer may intervene between the chip supporting the electrodes and an overlying structure, such as an expansion membrane, and the spacer's height defines the height of the electrolyte reservoir. In general, the spacer surrounds and is not bonded to the electrodes.

In another aspect, the invention pertains to a method of manufacturing a device for administering a liquid - in particular, a device comprising a housing and, within the housing, a pump assembly including a reservoir, a gas-driven forcing mechanism and a cannula for conducting liquid from the reservoir to an ejection site exterior to the housing in response to pressure applied by the forcing mechanism, and internal to the pumping mechanism, an electrolyte reservoir. In various embodiments, the method comprises the steps of providing at least two electrodes; masking a portion of at least one of the electrodes with a gas-impermeable material; and introducing the electrodes into the electrolyte reservoir, whereby an exposed portion of the electrodes is patterned to achieve a target operating ratio of gas recombination to gas generation in the reservoir.

The masking step may comprise depositing the gas-impermeable material onto at least one of the electrodes through a pattern template. Alternatively, the gas-impermeable material may be applied by chemical vapor deposition. In various embodiments, the masking step comprises depositing the gas-impermeable material onto at least one of the electrodes by pointwise printing in a predetermined pattern. Alternatively, the masking step may comprise adhering a gas-impermeable material onto at least one of the electrodes. A spacer may be incorporated onto a non-electrode portion of the electrolyte reservoir.

Reference throughout this specification to "one example," "an example," "one embodiment," or "an embodiment" means that a particular feature, structure, or characteristic described in connection with the example is included in at least one example of the present technology. Thus, the occurrences of the phrases "in one example," "in an example," "one embodiment," or "an embodiment" in various places throughout this specification are not necessarily all referring to the same example. Furthermore, the particular features, structures, routines, steps, or characteristics may be combined in any suitable manner in one or more examples of the technology. The headings provided herein are for convenience only and are not intended to limit or interpret the scope or meaning of the claimed technology. The term "substantially" or "approximately" means ±10% (e.g., by weight or by volume), and in some embodiments, ±5%. The term "consists essentially of" means excluding other materials that contribute to function, unless otherwise defined herein. Nonetheless, such other materials may be present, collectively or individually, in trace amounts.

### BRIEF DESCRIPTION OF THE DRAWINGS

In the drawings, like reference characters generally refer to the same parts throughout the different views. Also, the drawings are not necessarily to scale, with an emphasis instead generally being placed upon illustrating the principles of the invention. In the following description, various embodiments of the present invention are described with reference to the following drawings, in which:
FIG. 1 schematically illustrates a side-cross-sectional view of an electrolytic pump useful in conjunction with the invention.
FIG. 2 is a plan view of an electrolysis electrode arrangement suitable for use in the pump shown in FIG. 1.
FIG. 3 is a plan view of the electrode arrangement shown in FIG. 2, with a mask covering a portion of the electrode surfaces.
FIG. 4 is an exploded view showing use of a spacer along with the mask illustrated in FIG. 3.

### DETAILED DESCRIPTION

A typical drug-delivery device includes a reservoir, which contains a liquid comprising a therapeutic agent (e.g., a drug), and a cannula in fluid communication with the reservoir. At or near its distal end, the cannula has an outlet configured for fluid communication with a patient's target treatment site (e.g., the patient's eye, ear, brain, muscle, etc.). The device also includes a pair of electrodes in an electrolysis cell. A voltage applied between the electrodes produces gas from the electrolysis fluid. The produced gas exerts force on a force transducer such as a piston, diaphragm, or membrane, which forces the liquid to flow from the reservoir into the cannula and through the outlet. In other words, the electrodes operate an electrolytic pump. Various pressure transduction configurations and interfaces between the electrolysis reservoir and drug reservoir may be adapted to accommodate the structural limitations of the pump. These limitations are greater in embodiments where the drug-delivery devices are implantable. Alternative fluid communication methods including one or more cannulas, needles, permeable membranes, or sintering gradients may be incorporated according to the requirements of the target treatment site and therapeutic agent to be delivered.

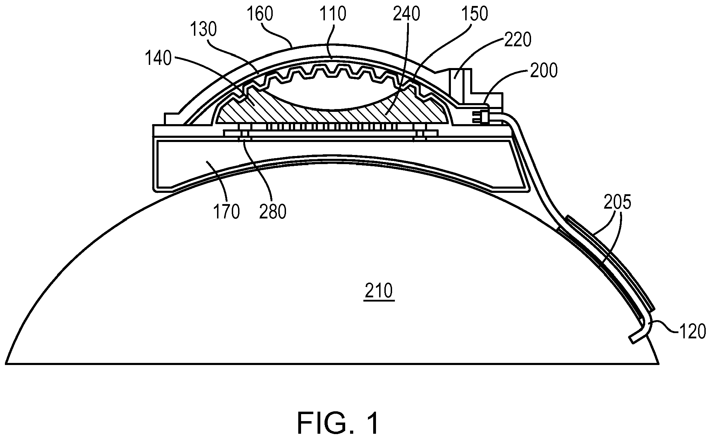

A representative electrolytically driven drug-delivery device 100 is shown in FIG. 1. The illustrated device 100 includes a pair of chambers 130, 140 (e.g., parylene envelopes) and a cannula 120. The top chamber 130 defines a drug reservoir that contains one or more drugs to be administered in liquid form, and the bottom chamber 140 contains a fluid (e.g., and electrolytic fluid) which, when subjected to electrolysis, evolves a gas including one or more gaseous products (e.g. in one embodiment, electrolysis of the fluid within the electrolysis chamber produces two gases, H₂ and O₂). The two chambers are separated by a diaphragm 150. The diaphragm 150 may be elastic and/or may be corrugated to provide for expansion thereof in response to the phase-change of the fluid within the bottom chamber 140 from a liquid to a gaseous state. The diaphragm 150 may be manufactured from one or more parylene films and/or a composite material. The diaphragm 150 and other components of the fluid pathway of the therapeutic agent may be made of biocompatible materials.

The chambers 130, 140 may be positioned within a shaped protective casing or shell 160 made of a relatively rigid biocompatible material (e.g., medical-grade polypropylene, a metal, and/or a biocompatible plastic). The shell 160 provides a hard surface against which an outer wall 110 of the drug reservoir chamber 130 exerts pressure and which protects the pump from inadvertent external forces. The shell 160 may include a solid, perforated or non-perforated biocompatible material coated in parylene. Control circuitry 170, including, for example, a battery and an induction coil for power and data transmission, are embedded under the bottom chamber 140 (e.g., between the bottom wall 280 of the bottom electrolysis chamber 140 and the floor of the shell 160). In one embodiment, the control circuitry 170 is embedded within a protective encapsulation such as, but not limited to, a silicon and/or parylene encapsulation. The control circuitry 170 provides power to one or more electrolysis electrodes 240 positioned within the bottom chamber 140, and may be secured to the electrolysis electrodes 240 by a material such as, but not limited to, a conductive epoxy including a biocompatible material (e.g. gold or silver). The electrolysis electrodes 240 may be formed on or within a parylene film forming the bottom surface of the electrolysis chamber 140. An adhesion layer (e.g. including or consisting of titanium) may be used to adhere the electrolysis electrodes 240 to a bottom surface of the electrolysis chamber 140. Alternatively, the bottom surface of the electrolysis chamber 140, to which the electrolysis electrodes 240 are coupled or embedded within, may include a substrate formed from a material including, but not limited to, alumina, zirconium oxide, ceramic, and/or sapphire. Activation of these electrolysis electrodes 240 produces a phase change in the electrolytic fluid within the bottom chamber 140 by evolving the fluid from a liquid to a gaseous state (i.e. generating a gas through electrolysis). The electrodes 240 generally act as (i.e., may contain or consist of) the recombination catalyst. Optionally, an additional recombination catalyst may be added to the electrolysis chamber to augment recombination.

The cannula 120 connects the drug chamber 130 with a treatment site. A check valve 200, one or more flow sensors 205, and/or one or more chemical or pressure sensors 205 may be positioned within the cannula 120 or internal to the shell 160 to control and/or monitor the flow of drug from the drug chamber 130, through the cannula 120, and into the treatment site. The treatment site may be an eye 210 of a patient, or may be any other target body portion. A hole may be formed through the protective shell 160 and a refill port 220 configured thereon.

Pumping action, including closed-loop operation, may be controlled by control circuitry 170. In one embodiment, an induction coil permits wireless (e.g., radio-frequency (RF)) communication with an external controller (e.g., a portable control handset), which may also be used, for example, to charge the battery of the control circuitry 170. The external controller may be used to send wireless signals to the control circuitry 170 in order to program, reprogram, operate, calibrate, or otherwise configure the operation of the pump 100. The control circuitry 170 may, for example, communicate electrically with the electrolysis electrodes 240 in the bottom electrolysis chamber 140 by means of metal interconnects 280 spanning the bottom wall of the electrolysis chamber 140. In one embodiment, the electrolysis electrodes 240 are platinum. Alternatively, any other appropriate conductive material (e.g., copper, gold, or silver on parylene, ceramic, or a biocompatible insulator) may be used.

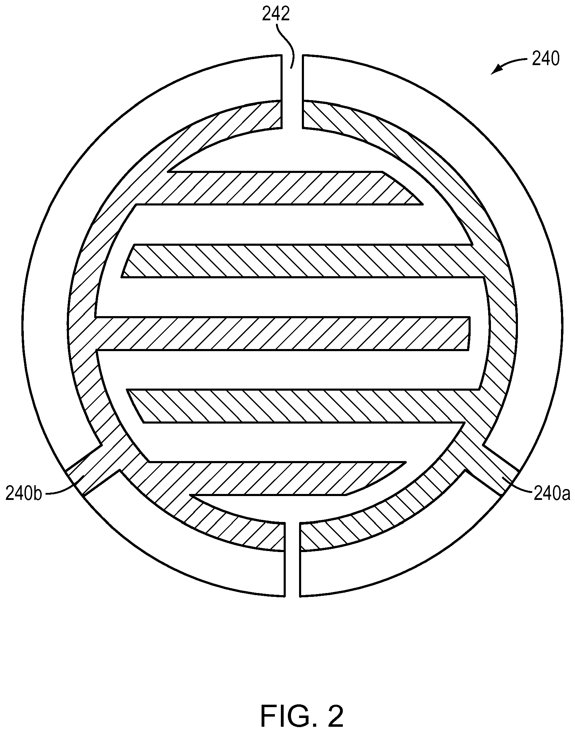

A representative configuration of the electrodes 240 is shown in FIG. 2. In this embodiment, the individual electrode elements 240a, 240b are interdigitated and may cover most of the floor of the elecrolysis chamber (for efficient use of limited space). It should be understood that the rendering is schematic and exemplary; the electrodes need not be interdigitated, and interdigitated electrodes need not conform to the simple illustrated pattern. In general, working embodiments will contain a greater number of interdigitations with less insulative surface area to optimize the efficiency of electrolysis gas generation and recombination and associated current and voltage requirements.

The insulative region 242 maintains the necessary galvanic separation between the electrodes 240a, 240b, which have tabs that allow for convenient connection to lead wires electrically connecting the electrodes to the control circuitry.

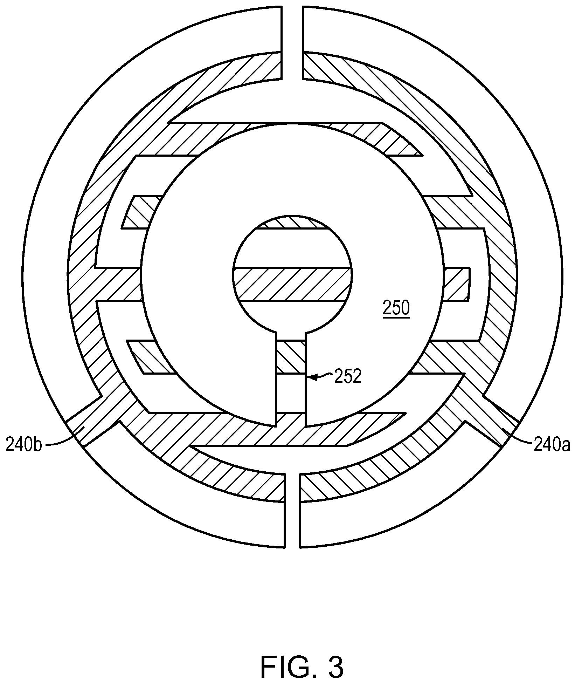

With reference to FIG. 3, a single mask 250 covers an annular portion of the interdigitated electrode pair 240; due to electrode symmetry, a proportionally equivalent area of both electrodes 240a, 240b will be masked off. FIG. 3 further shows that the single mask 250 that covers the annular portion of the interdigitated electrode pair 240 may optionally contain one or more alignment features 252. It is to be understood that various different electrode patterns create different axes of symmetry and that the mask shape, size, and quantity may be altered to accommodate the electrolysis gas recombination/generation ratio desired. That is, the ratio of masked to unmasked electrode regions may be chosen to achieve a target operating ratio of gas recombination to gas generation.

In various embodiments, the mask 250 is made of a solid, gas-impermeable material (e.g., a thermoplastic such as polyether ether ketone (PEEK) or a ceramic or other insulator) that is affixed to or deposited over the electrodes 240 to lower the recombination rate by reducing the exposed area of the electrodes in an electrolysis cell. The shape and surface area of the mask can be altered to tune electrolysis properties. For example, the geometry of a round electrode may be revised into another shape (e.g., a square), or an electrode pair may be patterned (e.g., with interdigitating fingers) in order to comparatively test the effect of shape and configuration on performance.

In general, the most important design parameter for a mask from a functional perspective is the surface area of the mask relative to the surface area of the electrodes. However, the shape of the mask also has an effect on the ratio of the gas-recombination rate to the gas-generation rate. The illustrated mask 250 has a ring shape. Experimentation has shown that on a circular set of electrodes, a mask with the same surface area but no center hole did not yield the recombination/generation ratio desired. This was due to the way this cell operated- specifically, generated gas moved towards the center of the electrode area. With a mask shaped as a solid disk, recombination would occur slowly because, with increasing inward distance from the disk edge, a gas bubble has farther to travel before clearing the mask and reaching the outer electrodes. With a hole in the center of the mask, by contrast, a moderate amount of recombination occurs in the center, and no gas bubble has further to travel than the annular extent of the mask.

In some embodiments, the electrolysis mask is attached to the chip by an adhesive, e.g., an epoxy. Suitable adhesives tolerate both electrolysis and recombination without excessive delamination, which may gradually modify the recombination/generation ratio as the mask fails over time. A rigid electrolysis mask material may be selected to minimize the delaminating effects of mechanical forces caused by the flexing of the electrolysis mask when electrolysis gas is generated. Once the optimal electrode pattern is established using the mask, it may be applied to finished chips; that is, the mask dictates which portions of the electrode pattern should be omitted in the finished production chip.

In other embodiments, the electrolysis mask is deposited, e.g., through a patterned stencil template. For example, materials such as silicon nitride can be deposited by chemical vapor deposition (CVD), though any deposition technique suited to the selected mask material can be used. With this approach, successive depositions can gradually widen the mask, with each deposition occupying an equivalent incremental area. The performance of the chip can be tested between depositions. Alternatively, the mask may be deposited by ink-jet or other pointwise deposition process in accordance with a digitally stored pattern.

Furthermore, mask deposition can be used to fabricate finished production electrolysis chips in addition to chips used for experimental purposes. For example, a basic template electrode pattern may be established for mass production, and this pattern may be tailored, using application-specific masks, to devices having different performance requirements. That is, masking may be used in the manufacture of finished devices in addition to its experimental use in defining an optimal electrode pattern for a particular chip.

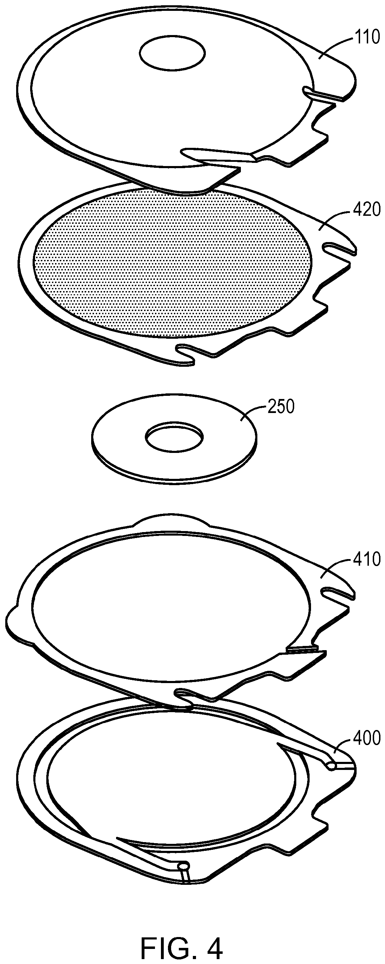

FIG. 4 shows an exploded schematic perspective view of the pump assembly. As described above, the recombination mask 250 overlies a portion of the electrodes (not shown in this figure) on the electrolysis chip 400. As described above, adhesive, deposition or other methods may be used to attach the electrolysis recombination mask 250 to the electrolysis chip 400. Optionally, a spacer 410 may also be attached to the electrolysis chip 400 to accommodate the height of the electrolysis recombination mask 250 relative to overlying components. In particular, a force transducer 420 - i.e., an expandable membrane as described in connection with FIG. 1 - overlies the electrode chip 400. The space between the electrode chip 400 and the force transducer 420 constitutes the volume of the electrolysis chamber. Consequently, the thickness of the spacer 410 can be varied to achieve a target chamber volume and, therefore, the amount of electrolysis fluid in the chamber. The spacer 410 may contain openings (e.g. pass-throughs, holes, slots, etc.) into which electrolysis fluid fill tubes of a specific height may be integrated to facilitate electrolyte solution fill or addition. In embodiments where the peripheral regions of the electrode are masked, the spacer may integrate the mask and be affixed to the electrolysis cell as one component.

Certain embodiments of the present invention have been described above. It is, however, expressly noted that the present invention is not limited to those embodiments, but rather the intention is that additions and modifications to what was expressly described herein are also included within the scope of the claims. As such, the invention is not to be defined only by the preceding illustrative description.

## Claims

1. A device (100) for administering a liquid, the device comprising:
a housing (160);
within the housing, a pump assembly including a reservoir (130), an electrolytic forcing mechanism (140, 240) and a cannula (120) for conducting liquid from the reservoir to an ejection site exterior to the housing in response to pressure applied by the forcing mechanism; and
internal to the forcing mechanism, an electrolyte reservoir (140) and, therein, an electrode assembly (240) comprising (i) at least two electrodes (240a, 240b) and (ii) over a portion of at least one of the electrodes, a gas-impermeable mask (250), wherein the mask is sized and shaped so that a ratio of masked to unmasked electrode portions achieves a target operating ratio of gas recombination to gas generation.

2. The electrolytic pump of claim 1, wherein the electrodes (240a, 240b) are disposed on an electrolysis chip (400).

3. The electrolytic pump of claim 1, wherein the electrodes (240a, 240b) comprise at least one of platinum, gold, or silver on parylene, ceramic, or a biocompatible insulator.

4. The electrolytic pump of claim 1, wherein the electrodes (240a, 240b) act as a recombination catalyst and further comprising an additional recombination catalyst.

5. The device of claim 1, wherein at least a portion of the mask (250) structure is bonded to the electrodes with an epoxy.

6. The device of claim 1, wherein the mask (250) structure comprises PEEK, ceramic, aluminum.

7. The device of claim 1, further comprising a spacer (410) having a height that defines a height of the electrolyte reservoir (140).

8. The device of claim 7, wherein the spacer (410) surrounds and is not bonded to the electrodes (240a, 240b).

9. A method of manufacturing a device (100) for administering a liquid, the device comprising a housing (160) and, within the housing, a pump assembly including a reservoir (130), a gas-driven forcing mechanism (140, 240) and a cannula (120) for conducting liquid from the reservoir to an ejection site exterior to the housing in response to pressure applied by the forcing mechanism, and internal to the pumping mechanism, an electrolyte reservoir (140), the method comprising the steps of:
providing at least two electrodes (240a, 240b);
masking a portion of at least one of the electrodes with a gas-impermeable material (250); and
introducing the electrodes (240a, 240b) into the electrolyte reservoir (140), whereby an exposed portion of the electrodes is patterned to achieve a target operating ratio of gas recombination to gas generation in the reservoir.

10. The method of claim 9, wherein the masking step comprises depositing the gas-impermeable material (250) onto at least one of the electrodes (240a, 240b) through a pattern template.

11. The method of claim 10, wherein the gas-impermeable material (250) is applied by chemical vapor deposition.

12. The method of claim 9, wherein the masking step comprises depositing the gas-impermeable material (250) onto at least one of the electrodes (240a, 240b) by pointwise printing in a predetermined pattern.

13. The method of claim 9, wherein the masking step comprising adhering a gas-impermeable material (250) onto at least one of the electrodes (240a, 240b).

14. The method of claim 9, further comprising the step of incorporating a spacer (410) onto a non-electrode portion of the electrolyte reservoir (140).

## Patentansprüche

1. Vorrichtung (100) zum Verabreichen einer Flüssigkeit, wobei die Vorrichtung umfasst:
ein Gehäuse (160);
innerhalb des Gehäuses eine Pumpenbaugruppe mit einem Reservoir (130), einem elektrolytischen Druckmechanismus (140, 240) und einer Kanüle (120) zum Leiten von Flüssigkeit aus dem Reservoir zu einer Ausstoßstelle außerhalb des Gehäuses als Antwort auf durch den Druckmechanismus ausgeübten Druck; und
innerhalb des Druckmechanismus ein Elektrolytreservoir (140) und darin eine Elektrodenbaugruppe (240), die (i) mindestens zwei Elektroden (240a, 240b) und (ii) auf einem Abschnitt mindestens einer der Elektroden eine gasundurchlässige Maske (250) umfasst, worin die Maske so bemessen und geformt ist, dass ein Verhältnis von maskierten zu unmaskierten Elektrodenabschnitten ein Sollbetriebsverhältnis von Gasrekombination zu Gaserzeugung erzielt.

2. Elektrolytische Pumpe nach Anspruch 1, worin die Elektroden (240a, 240b) auf einem Elektrolysechip (400) angeordnet sind.

3. Elektrolytische Pumpe nach Anspruch 1, worin die Elektroden (240a, 240b) mindestens eines von Platin, Gold oder Silber auf Parylen, Keramik oder einem biologisch verträglichen Isolator umfassen.

4. Elektrolytische Pumpe nach Anspruch 1, worin die Elektroden (240a, 240b) als Rekombinationskatalysator wirken und ferner einen zusätzlichen Rekombinationskatalysator umfassen.

5. Vorrichtung nach Anspruch 1, worin mindestens ein Teil der Maskenstruktur (250) mit einem Epoxidharz auf die Elektroden geklebt ist.

6. Vorrichtung nach Anspruch 1, worin die Struktur der Maske (250) PEEK, Keramik, Aluminium umfasst.

7. Vorrichtung nach Anspruch 1, ferner umfassend einen Abstandshalter (410) mit einer Höhe, die eine Höhe des Elektrolytreservoirs (140) definiert.

8. Vorrichtung nach Anspruch 7, worin der Abstandshalter (410) die Elektroden (240a, 240b) umgibt und nicht mit ihnen verklebt ist.

9. Verfahren zum Herstellen einer Vorrichtung (100) zum Verabreichen einer Flüssigkeit, wobei die Vorrichtung umfasst: ein Gehäuse (160) und, innerhalb des Gehäuses, eine Pumpenbaugruppe mit einem Reservoir (130), einem gasbetriebenen Druckmechanismus (140, 240) und einer Kanüle (120) zum Leiten von Flüssigkeit aus dem Reservoir zu einer Ausstoßstelle außerhalb des Gehäuses als Antwort auf durch den Druckmechanismus ausgeübten Druck, und innerhalb des Druckmechanismus ein Elektrolytreservoir (140), wobei das Verfahren die Schritte umfasst:
Bereitstellen von mindestens zwei Elektroden (240a, 240b);
Maskieren eines Abschnitts von mindestens einer der Elektroden mit einem gasundurchlässigen Material (250); und
Einführen der Elektroden (240a, 240b) in das Elektrolytreservoir (140), wodurch ein freiliegender Teil der Elektroden dafür strukturiert ist, ein Sollbetriebsverhältnis von Gasrekombination zu Gaserzeugung im Reservoir zu erzielen.

10. Verfahren nach Anspruch 9, worin der Maskierungsschritt umfasst: Abscheiden des gasundurchlässigen Materials (250) auf mindestens einer der Elektroden (240a, 240b) durch eine Strukturschablone.

11. Verfahren nach Anspruch 10, worin das gasundurchlässige Material (250) durch chemische Gasphasenabscheidung aufgebracht wird.

12. Verfahren nach Anspruch 9, worin der Maskierungsschritt umfasst: Abscheiden des gasundurchlässigen Materials (250) auf mindestens einer der Elektroden (240a, 240b) durch punktweises Drucken in einer vorbestimmten Struktur.

13. Verfahren nach Anspruch 9, worin der Maskierungsschritt umfasst: Ankleben eines gasundurchlässigen Materials (250) auf mindestens einer der Elektroden (240a, 240b).

14. Verfahren nach Anspruch 9, ferner den Schritt umfassend: Einbauen eines Abstandshalters (410) auf einem Nicht-Elektrodenabschnitt des Elektrolytreservoirs (140).

## Revendications

1. Dispositif (100) permettant d'administrer un liquide, le dispositif comprenant :
un logement (160) ;
au sein du logement, un ensemble pompe comprenant un réservoir (130), un mécanisme de forçage électrolytique (140, 240) et une canule (120) permettant d'acheminer du liquide en provenance du réservoir vers un site d'éjection extérieur à le logement en réaction à une pression appliquée par le mécanisme de forçage ; et
à l'intérieur du mécanisme de forçage, un réservoir d'électrolyte (140) et, en son sein, un ensemble électrode (240) comprenant (i) au moins deux électrodes (240a, 240b) et (ii) sur une partie d'au moins une des électrodes, un masque imperméable au gaz (250), dans lequel le masque est dimensionné et formé de sorte qu'un rapport des parties électrode masquées sur les parties électrode non masquées atteint un rapport de fonctionnement cible de recombinaison de gaz sur génération de gaz.

2. Pompe électrolytique selon la revendication 1, dans laquelle les électrodes (240a, 240b) sont fournies sur une puce d'électrolyse (400).

3. Pompe électrolytique selon la revendication 1, dans laquelle les électrodes (240a, 240b) comprennent au moins un parmi platine, or, ou argent sur parylène, céramique, ou un insolant biocompatible.

4. Pompe électrolytique selon la revendication 1, dans laquelle les électrodes (240a, 240b) font office de catalyseur de recombinaison et comprennent en outre un catalyseur de recombinaison supplémentaire.

5. Dispositif selon la revendication 1, dans lequel au moins une partie de la structure du masque (250) est liée aux électrodes grâce à une résine époxy.

6. Dispositif selon la revendication 1, dans lequel la structure du masque (250) comprend PEEK, céramique, aluminium.

7. Dispositif selon la revendication 1, comprenant en outre une cale d'espacement (410) présentant une hauteur qui définit une hauteur du réservoir d'électrolyte (140).

8. Dispositif selon la revendication 7, dans lequel la cale d'espacement (410) entoure les électrodes (240a, 240b) et n'est pas liée à celles-ci.

9. Procédé de fabrication d'un dispositif (100) permettant d'administrer un liquide, le dispositif comprenant un logement (160) et, au sein du logement, un ensemble pompe comprenant un réservoir (130), un mécanisme de forçage par poussée de gaz (140, 240) et une canule (120) permettant d'acheminer du liquide en provenance du réservoir vers un site d'éjection extérieur du logement en réaction à une pression appliquée par le mécanisme de forçage, et, à l'intérieur du mécanisme de pompage, un réservoir d'électrolyte (140), le procédé comprenant les étapes consistant à :
fournir au moins deux électrodes (240a, 240b) ;
masquer une partie d'au moins une des électrodes avec un matériau imperméable aux gaz (250) ; et
introduire les électrodes (240a, 240b) dans le réservoir d'électrolyte (140), grâce à quoi une partie exposée des électrodes est dessinée de manière à atteindre un rapport de fonctionnement cible de recombinaison de gaz sur génération de gaz au sein du réservoir.

10. Procédé selon la revendication 9, dans lequel l'étape de masquage comprend une étape consistant à déposer le matériau imperméable aux gaz (250) sur au moins une des électrodes (240a, 240b) à travers un gabarit de dessin.

11. Procédé selon la revendication 10, dans lequel le matériau imperméable aux gaz (250) est appliqué par dépôt chimique en phase vapeur.

12. Procédé selon la revendication 9, dans lequel l'étape de masquage comprend une étape consistant à déposer le matériau imperméable aux gaz (250) sur au moins une des électrodes (240a, 240b) grâce à une étape consistant à imprimer point par point selon un modèle prédéterminé.

13. Procédé selon la revendication 9, dans lequel l'étape de masquage comprend une étape consistant à coller un matériau imperméable aux gaz (250) sur au moins une des électrodes (240a, 240b).

14. Procédé selon la revendication 9, comprenant en outre l'étape consistant à incorporer une cale d'espacement (410) sur une partie non électrode du réservoir d'électrolyte (140).
